# EUROPÄISCHE PATENTSCHRIFT

(11) **EP 1 100 767 B1**
(45) Veröffentlichungstag und Bekanntmachung des Hinweises auf die Patenterteilung: **24.07.2002**
(21) Anmeldenummer: 99938314.4
(22) Anmeldetag: 22.07.1999
(51) Int. Cl.: C07C 217/60, A61K 31/135

(54) **PHENYLETHYLAMINDERIVATE, VERFAHREN ZU IHRER HERSTELLUNG UND IHRE VERWENDUNG ALS ARZNEIMITTEL**
NOVEL PHENYLETHYLAMINE DERIVATIVES, A METHOD FOR THE PRODUCTION THEREOF AND THEIR USE AS MEDICAMENTS
NOUVEAUX DERIVES PHENYLETHYLAMINES, LEURS PROCEDES DE PREPARATION ET LEUR UTILISATION COMME MEDICAMENT

(30) Priorität: 31.07.1998 DE 19834713
(43) Veröffentlichungstag der Anmeldung: 23.05.2001
(73) Patentinhaber: Boehringer Ingelheim Pharma KG, 55216 Ingelheim am Rhein (DE)
(72) Erfinder: ANDERSKEWITZ, Ralf, D-55411 Bingen (DE); BIRKE, Franz, D-55218 Ingelheim am Rhein (DE); JENNEWEIN, Hans-Michael, D-65193 Wiesbaden (DE)
(74) Vertreter: Laudien, Dieter, Dr.
(86) Internationale Anmeldenummer: EP9905221
(87) Internationale Veröffentlichungsnummer: WO0007977

(56) Entgegenhaltungen:
- WO-A-96/11192
- WO-A-98/49131
- DE-A- 4 424 713
- DE-A- 4 424 714

## Beschreibung

Die vorliegende Erfindung betrifft neue Phenylethylaminderivate, Verfahren zu ihrer Herstellung sowie ihre Verwendung als Arzneimittel. Die erfindungsgemäßen Phenylethylaminderivate entsprechen der allgemeinen Formel 1 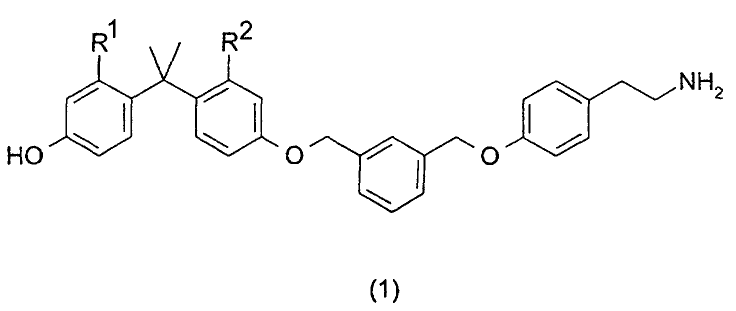 in der
- R¹ und R²: unabhängig voneinander Wasserstoff oder Fluor
bedeuten können
in Form der freien Basen oder der entsprechenden Säureadditionssalze mit pharmakologisch unbedenklichen Säuren.

Bevorzugt sind folgende zwei Verbindungen: 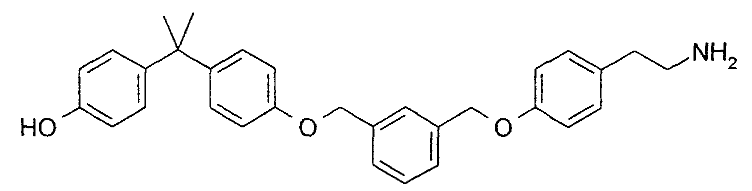 und

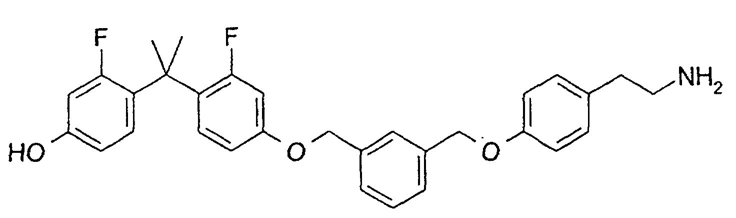

Wie gefunden wurde, zeichnen sich die erfindungsgemäßen Verbindungen der Formel I durch vielfältige Anwendungsmöglichkeiten auf therapeutischem Gebiet sowie durch ihre orale Wirksamkeit aus. Hervorzuheben sind solche Anwendungsmöglichkeiten, für welche die LTB₄-rezeptorantagonistischen Eigenschaften eine Rolle spielen. Hier sind insbesondere zu nennen:

Arthritis, Asthma, chronische obstruktive Lungenerkrankungen, etwa chronische Bronchitis, α₁-Antitrypsin-Mangel, Psoriasis, Colitis ulcerosa, entzündliche Darmerkrankungen, durch nichtsteroidale Antiphlogistika induzierte Gastro- oder Enteropathie, cystische Fibrose, Alzheimer-Krankheit, Schock, Reperfusionsschäden/Ischämien, Atherosklerose, Multiple Sklerose.

Auch lassen sich mit den neuen Verbindungen Krankheiten oder Zustände behandeln, bei denen die Passage von Zellen aus dem Blut über das vaskuläre Endothelium in das Gewebe von Bedeutung ist (etwa Metastasis) oder Krankheiten und Zustände, bei denen die Kombination des LTB₄ oder eines anderen Moleküls (beispielsweise 12-HETE) mit dem LTB₄-Rezeptor einen Einfluß auf die Zell-Proliferation hat (etwa chronische myelozytische Leukämie).

Die neuen Verbindungen können auch in Kombination mit anderen Wirkstoffen angewendet werden, etwa solchen, die für dieselben Indikationen Verwendung finden, oder z.B. mit Antiallergika, Sekretolytika, β₂-Adrenergika, inhalativ und oral anwendbaren Steroiden, Antihistaminika, PAF-Antagonisten, NSAID sowie Glucocorticoide. Die Verabreichung kann topisch, oral, transdermal, nasal, parenteral oder inhalativ erfolgen.

Zur pharmakologischen und biochemischen Untersuchung der Wirkungsverhältnisse eigenen sich Tests, wie sie beispielsweise in der WO 93/16036, S. 15 bis 17 ("Mäuseohrtest")- auf die hier inhaltlich Bezug genommen wird - dargestellt sind.

Die erfindungsgemäßen Verbindungen erweisen sich in vitro sowie in vivo als besonders hochpotente LTB₄-Antagonisten. Die beiden erfindungsgemäßen Substanzen zeigen eine hochaffine Bindung zum LTB₄-Rezeptor. Überdies weisen sie im oben angeführten Mäuseohrtest nach oraler Gabe ED₅₀-Werte von 0,02 bzw. 0,03 mg/kg auf.

Die therapeutische oder prophylaktische Dosis ist - außer von der Wirkungsstärke der einzelnen Verbindungen und dem Körpergewicht des Patienten - abhängig von der Beschaffenheit und Ernsthaftigkeit des Krankheitszustandes. Bei oraler Anwendung liegt die Dosis zwischen 1 und 500 mg, vorzugsweise zwischen 20 und 250 mg. Bei inhalativer Anwendung werden dem Patienten zwischen etwa 0,5 und 25, vorzugsweise zwischen etwa 2 und 20 mg Wirkstoff zugeführt.

Inhalationslösungen enthalten im allgemeinen zwischen etwa 0,5 und 5 % Wirkstoff. Die neuen Verbindungen können in üblichen Zubereitungen verabreicht werden, etwa als Tabletten, Dragées, Kapseln, Oblaten, Pulver, Granulate, Lösungen, Emulsionen, Sirupe, Inhalationsaerosole, Salben, Suppositorien.

Die nachstehenden Beispiele zeigen einige Möglichkeiten für die Formulierung der Darreichungsformen:

### Formulierungsbeispiele

### 1. Tabletten

a) Hergestellt gemäß Pluronic-Schmelz-Einbettung

| | | |
|---|---|---|
| Gehalt | 1 % | 10 % |
| Wirkstoff gemäß Formel 1 | 1 mg | 10 mg |
| Poloxamer NF | 99 mg | 90 mg |

b) Wettability improved Formulierung

| Bestandteil | Gehalt | Gehalt |
|---|---|---|
| Wirkstoff gemäß Formel 1 | 25,00 mg | 75,00 mg |
| Mikrokristalline Cellulose | 22,75 mg | 68,25 mg |
| Laktose Monohydrat | 22,75 mg | 68,25 mg |
| Methylcellulose | 1,25 mg | 3,75 mg |
| Natriumlaurylsulfat | 0,25 mg | 0,75 mg |
| Crospovidon(e) | 2,25 mg | 6,75 mg |
| Magnestiumstearat | 0,75 mg | 2,25 mg |
| Summe | 75,00 mg | 225 mg |

### 2. Suppositorien

| Zusammensetzung: | |
|---|---|
| Wirkstoff gemäß der Erfindung | 100 Gew.-Teile |
| Laktose, gepulvert | 45 Gew.-Teile |
| Kakao-Butter | 1555 Gew.-Teile |

Die Bestandteile werden in üblicher Weise zu Suppositorien von 1,7 g Gewicht verarbeitet.

### 3. Inhalationspulver

Mikronisiertes Wirkstoffpulver (Verbindung der Formel I; Teilchengröße ca. 0,5 bis 7 µm) werden in einer Menge von 5 mg gegebenenfalls unter Zusatz mikronisierter Lactose in Hartgelatinekapseln abgefüllt. Das Pulver wird aus üblichen Inhalationsgeräten, z.B. gemäß der Deutsche Offenlegungsschrift Nr.: 33 45 722, auf die hiermit inhaltlich Bezug genommen wird, inhaliert.

Die neuen Verbindungen können nach an sich aus dem Stand der Technik bekannten Verfahren hergestellt werden. Dazu kommen insbesondere folgende drei Verfahren vorteilhafterweise in Betracht:

### 1. Umsetzung des geschützten Phenols der allgemeinen Formel 2

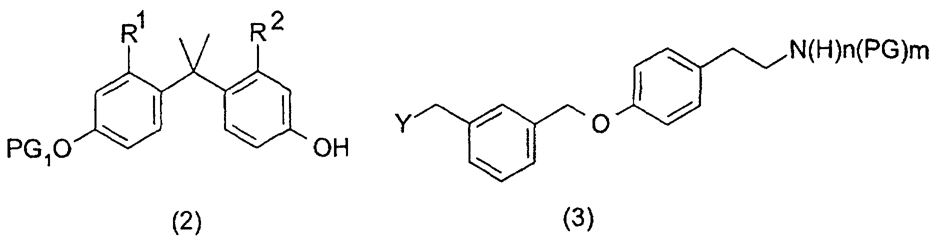

mit einem Ethylaminderivat der allgemeinen Formel 3
in denen
- PG₁: eine zum Schutz von Phenolen geeignete Schutzgruppe oder aber auch Wasserstoff;
- PG: eine zum Schutz von Aminen geeignete darstellen kann, wobei n Null oder1 und m eine der ganzen Zahlen 1 oder 2 bedeuten kann;
- Y: Fluor, Chlor, Brom oder Jod oder einen C1-C4-Alkyl- oder einen Arylsulfonatrest
bedeuten können und R¹ sowie R² die eingangs genannte Betdeutung haben.

Die Verbindungen werden mit basischen Hilfsmitteln wie z.B. Alkali- oder Eralkalihydroxiden, Alkali- oder Erdalkalicarbonaten, C₁-C₄-Akalialkoholaten in unter den gewählten Reaktionsbedingungen inerten Lösungsmitteln wie Formamiden - bevorzugt Dimethylfomamid (DMF), C₁-C₄-Alkylnitrilen- bevorzugt Acetonitril -, C₁-C₄-Alkylestem von Carbonsäuren - bevorzugt Essigsäureethylester oder Ameisensäureethylester -, aromatischen oder aliphatischen Kohlenwasserstoffen - bevorzugt Toluol - oder in verzweigten oder unverzweigten C₁-C₄-Alkoholen umgesetzt.

Im nachfolgenden Reaktionsschritt erfolgt die Abspaltung der Schutzgruppen insbesondere mit anorganischen oder organischen Säuren in geeigneten Solventien oder auf dem Wege der Hydrogenolyse bzw. oder mit anderen aus dem Stand der Technik bekannten Verfahren, die üblicherweise zur Abspaltung spezifischer Schutzgruppen verwendet werden.

### 2. Umsetzung zweier Verbindungen der allgemeinen Formel 4 und 5:

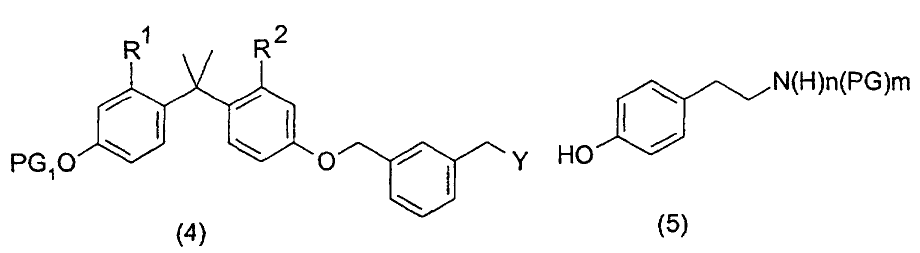

worin
- PG₁: Wasserstoff oder eine zum Schutz von für Phenolen geeignete Schutzgruppe;
- PG: eine zum Schutz von Aminen geeignete Schutzgruppe geeignete Schutzgruppe darstellen kann, wobei n Null oder1 und m eine der ganzen Zahlen 1 oder 2 bedeuten kann;
- Y: Fluor, Chlor, Brom oder Jod oder einen C₁-C₄-Alkyl- oder einen Arylsulfonatrest
bedeuten können und R¹ sowie R² die eingangs genannte Betdeutung haben.

Die Verbindungen werden mit basischen Hilfsmitteln wie z.B. Alkali- oder Eralkalihydroxiden, Alkali- oder Erdalkalicarbonaten, C₁-C₄-Akalialkoholaten in - unter den gewählten Reaktionsbedingungen inerten - Lösungsmitteln wie Formamiden - bevorzugt Dimethylfomamid (DMF), C₁-C₄-Alkylnitrilen - bevorzugt Acetonitril -, C₁-C₄-Alkylestern von Carbonsäuren - bevorzugt Essigsäureethylester oder Ameisensäureethylester -, aromatischen oder aliphatischen Kohfenwasserstoffen - bevorzugt Toluol - oder in verzweigten oder unverzweigten C₁-C₄-Alkoholen umgesetzt.

Im nachfolgenden Reaktionsschritt erfolgt die Abspaltung der Schutzgruppen insbesondere mit anorganischen oder organischen Säuren in geeigneten Solventien oder auf dem Wege der Hydrogenolyse oder mit anderen aus dem Stand der Technik bekannten Verfahren, die üblicherweise zur Abspaltung spezifischer Schutzgruppen verwendet werden.

### 3. Reduktion des Nitrostyrols (6)

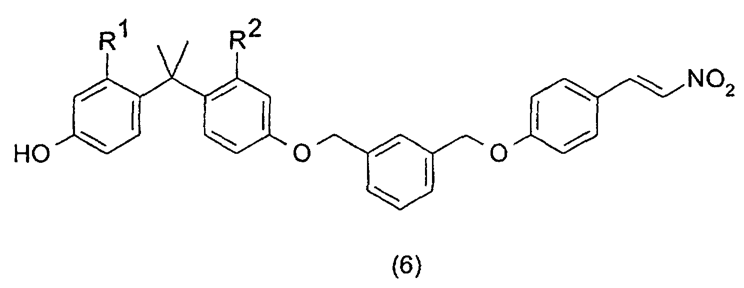

Die erfindungsgemäßen Verbindungen können durch Reduktion des Nitrostyrols (6) durch heterogene oder homogene Hydrierung mit Hilfe geeigneter Katalysatoren, mit Hilfe komplexer Hydride oder mit Boranen in geeignteten Lösungsmitteln hergestellt werden.

So kann die Reduktion beispielsweise in einem Solvenz aus der Gruppe Methanol, Ethanol oder in einem höheren Alkohol, DMF oder Wasser in Gegenwart eines Katalysators aus der Gruppe Raney-Nickel, Pd/C, Platin oder mit Hydrid-Reagenzien - insbesondere komplexen Hydriden aus der Gruppe NaBH₄, Ca(BH₄)₂, LiAlH₄ oder anderen Aluminium- oder Bor-Hydriden - bei Temperaturen in einem Intervall von 0 bis 100 °C und unter einem Wasserstoffdruck von mindestens 760 Torr (1,013bar) zum entsprechenden Amin der allgemeinen Formel 1 erfolgen.

Die oben in der Beschreibung der Herstellverfahren erwähnten Schutzgruppen sowie die Verfahrensbedingungen, die Schutz der phenolischen Hydroxylgruppen bzw.- der sekundären oder primäen Amine eingehalten werden müssen sowie deren nachträgliche Abspaltung sind aus dem Stand der Technik hinlänglich bekannt [T.W. Greene, P.G. Wuts, Protective Groups in Organic Synthesis. 2. Aufl., J. Wiley NCH, New York 1991].

Ebenso sind die Bedingungen zur Reduktion von Nitroverbindungen und die dazu erforderlichen Reduktionsmittel aus dem Stand der Technik hinlänglich bekannt [J. March: Advanced Organic Chemistry. Reactions, Mechanisms, and Structure. 4. Aufl., Wiley, New York 1992 und zit Lit.].

Verschiedenartige, andere Ausgestaltungen der Verfahren werden für den Fachmann aus der vorliegenden Beschreibung ersichtlich. Es wird jedoch ausdrücklich darauf hingewiesen, daß diese Beispiele und die diesen zugeordnete Beschreibung lediglich zum Zweck der Erläuterung vorgesehen und nicht als Einschränkung der Erfindung anzusehen sind.

### Synthesebeispiel

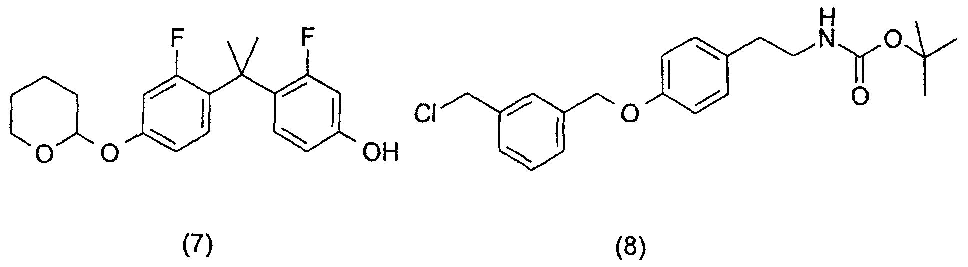

3,8 g des THP-geschützten Ethers 7, 2,7 g des BOC-geschützten Amins 8 und 1,5 g Kaliumcarbonat (K₂CO₃) werden in 100 ml Acetonitril über einen Zeitraum von 5 h am Rückfluß gekocht. Anschließend wird das Reaktionsmedium/Solvens abdestilliert. Der Rückstand wird zwischen Wasser und Essigsäureethylester verteilt. Die organische Phase wird abgetrennt und über Natriumsulfat (Na₂SO₄) getrocknet . Nach Abfiltrieren des Trockenmittels wird die Lösung eingedampft und der Rückstand chromatographisch mit Toluol/Aceton 95:5 über Kieselgel gereinigt. Ausbeute: 2,3 g.

Das Produkt wird in 20 ml Essigsäureethylester und 10 ml einer 3 M Lösung von HCl in Essigsäureethylester gelöst und 12 Std. bei Raumtemperatur gerührt. Die ausgefallenen Kristalle werden in wenig Methanol gelöst, und das Produkt mit Ether gefällt. Nach Absaugen und Trocknen erhält man 1 g der Verbindung (1), R=F, 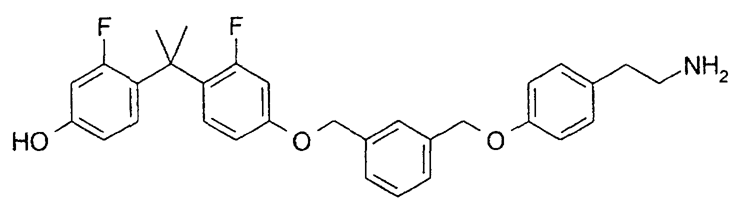 vom Schmelzpunkt 173°C.

Die Verbindung (1), R=H, wird auf analoge Weise hergestellt; Smp.: 182-185°C.

## Patentansprüche

1. Phenylethylaminderivate entsprechen der allgemeinen Formel I 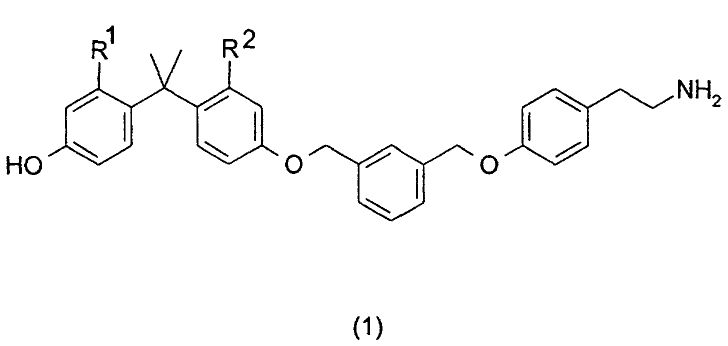 in der
R¹ und R² unabhängig voneinander Wasserstoff oder Fluor bedeuten können
in Form der freien Basen oder der enstsprechenden Säureadditionssalze mit pharmakologisch unbedenklichen Säuren.

2. Phenylethylethylaminderivat nach Anspuch 1 mit der Formel 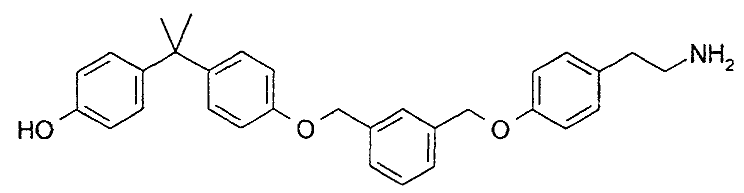 in Form der freien Basen oder der enstsprechenden Säureadditionssalze mit pharmakologisch unbedenklichen Säuren.

3. Phenylethylethylaminderivat nach Anspuch 1 mit der Formel 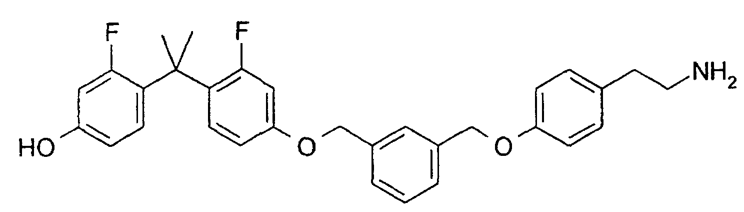 in Form der freien Basen oder der enstsprechenden Säureadditionssalze mit pharmakologisch unbedenklichen Säuren.

4. Verfahren zur Herstellung von Verbindungen der allgemeinen Formel 1, **dadurch gekennzeichnet, daß** man eine Verbindung der Formel (2) 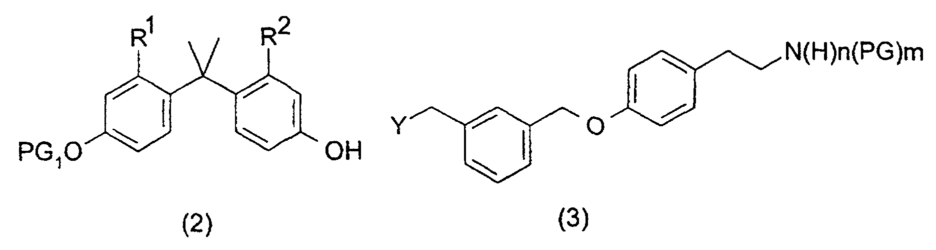 mit Ethylaminderivat der allgemeinen Formel 3, in denen
PG₁ eine zum Schutz von Phenolen geeignete Schutzgruppe oder Wasserstoff;
PG eine zum Schutz von Aminen geeignete Schutzgruppe geeignete Schutzgruppe darstellen kann, wobei n Null oder1 und m eine der ganzen Zahlen 1 oder 2 bedeuten kann;
Y Fluor, Chlor, Brom oder Jod oder einen C1-C4-Alkyl- oder einen Arylsulfonatrest
bedeuten können und R¹ sowie R² die eingangs genannte Betdeutung haben
in Gegenwart einer Hilfsbase aus der Gruppe der Alkali- oder Eralkalihydroxide, Alkali- oder Erdalkalicarbonate, C₁-C₄-Akalialkoholate in unter den gewählten Reaktionsbedingungen inerten Lösungsmitteln wie Formamiden - bevorzugt Dimethylfomamid (DMF), C₁-C₄-Alkylnitrilen - bevorzugt Acetonitril -, C₁-C₄-Alkylestern von Carbonsäuren - bevorzugt Essigsäureethylester oder Ameisensäureethylester -, aromatischen oder aliphatischen Kohlenwasserstoffen - bevorzugt Toluol - oder in verzweigten oder unverzweigten C₁-C₄-Alkoholen umsetzt, im nachfolgenden Reaktionsschritt die Schutzgruppen mit anderen aus dem Stand der Technik bekannten Verfahren abspaltet und das Reaktionsprodukt isoliert.

5. Verfahren zur Herstellung von Verbindungen der allgemeinen Formel 1, **dadurch gekennzeichnet, daß** man eine Verbindung der allgemeinen Formel 4 mit einer Verbindung der Formel 5 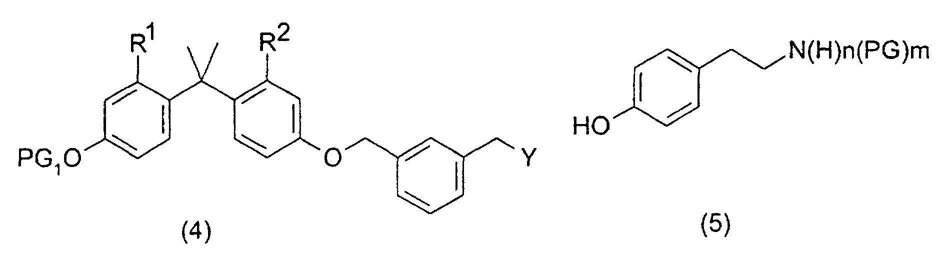
PG₁ Wasserstoff oder eine zum Schutz von für Phenolen geeignete Schutzgruppe;
PG eine zum Schutz von Aminen geeignete Schutzgruppe geeignete Schutzgruppe darstellen kann, wobei n Null oder 1 und m eine der ganzen Zahlen 1 oder 2 bedeuten kann;
Y Fluor, Chlor, Brom oder Jod oder einen C₁-C₄-Alkyl- oder einen Arylsulfonatrest
bedeuten können und R¹ sowie R² die eingangs genannte Betdeutung haben;
in Gegenwart einer Hilfsbase aus der Gruppe der Alkali- oder Eralkalihydroxide, Alkali- oder Erdalkalicarbonate, C₁-C₄-Akalialkoholate in - unter den gewählten Reaktionsbedingungen inerten - Lösungsmitteln wie Formamiden - bevorzugt Dimethylfomamid (DMF), C₁-C₄-Alkylnitrilen - bevorzugt Acetonitril -, C₁-C₄-Alkylestern von Carbonsäuren - bevorzugt Essigsäureethylester oder Ameisensäureethylester-, aromatischen oder aliphatischen Kohlenwasserstoffen - bevorzugt Toluol - oder in verzweigten oder unverzweigten C₁-C₄-Alkoholen umsetzt und im nachfolgenden Reaktionsschritt die Schutzgruppen - bevorzugt mit anorganischen oder organischen Säuren in geeigneten Solvenzien oder auf dem Wege der Hydrogenolyse mittels an sich bekannten Verfahren abspaltet.

6. Verfahren zur Herstellung von Verbindungen der allgemein Formel 1, **dadurch gekennzeichnet, daß**, man ein Nitrostyrol der allgemeinen Formel 6 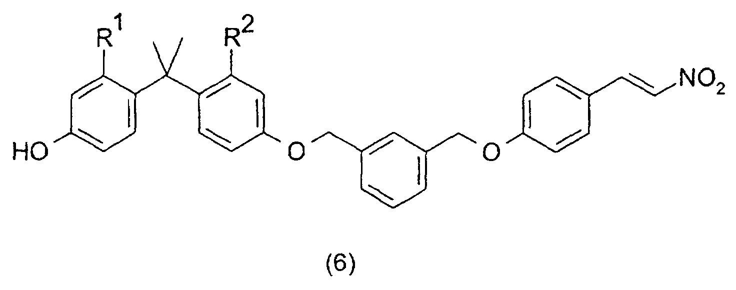 in einem Solvenz aus der Gruppe Methanol, Ethanol oder in einem höheren Alkohol, DMF oder Wasser in Gegenwart eines Katalysators aus der Gruppe Raney-Nickel, Pd/C, Platin oder mit Hydrid-Reagenzien - insbesondere komplexen Hydriden aus der Gruppe NaBH₄, Ca(BH₄)₂, LiAlH₄ oder anderen Aluminium- oder Bor-Hydriden - bei Temperaturen in einem Intervall von 0 bis 100 °C und unter einem Wasserstoffdruck von größer 760 Torr (1,013 bar) zum entsprechenden Amin der allgemeinen Formel 1 reduziert.

7. Pharmazeutische Zubereitung enthalten eine Verbindung der allgemeinen Formel 1 gemäß einem der Ansprüche 1,2 oder 3 und deren Säureadditionssalze neben üblichen Hilfs- und Trägersstoffen.

8. Verbindung der Formel 1 nach einem der Ansprüche 1, 2 oder 3 zur Verwendung als Arzneimittel.

9. Verbindung der Formel 1 nach einem der Ansprüche 1, 2 oder 3 zur Verwendung als Arzneimittel mit LTB₄-antagonistischer Wirkung.

10. Verwendung von Verbindungen der allgemeinen Formel I, deren Stereoisomeren sowie deren Säureadditionssalzen zur Herstellung eines Medikaments zur therapeutischen Behandlung von Arthritis, Asthma, chronischer obstruktiver Lungenerkrankung wie chronischer Bronchitis, Psoriasis, Colitis ulcerosa, durch nichtsteroidale Antiphlogistika induzierter Gastro- oder Enteropathie, cystischer Fibrose, Alzheimer-Krankheit, Schock, Reperfusionsschäden/Ischämien, Atherosklerose, multipler Sklerose.

## Claims

1. Phenylethylamine derivatives corresponding to general formula 1 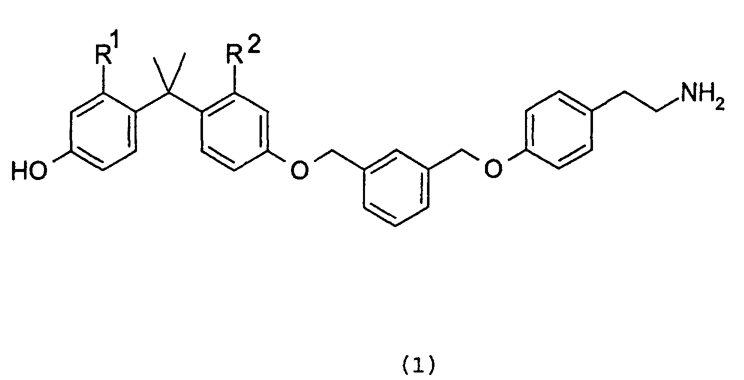 wherein
R¹ and R² independently of one another may denote hydrogen or fluorine
in the form of the free bases or the corresponding acid addition salts with pharmacologically acceptable acids.

2. Phenylethylamine derivative according to claim 1 of the formula 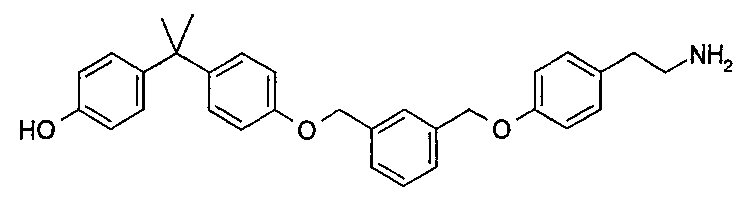 in the form of the free bases or the corresponding acid addition salts with pharmacologically acceptable acids.

3. Phenylethylamine derivative according to claim 1 of the formula 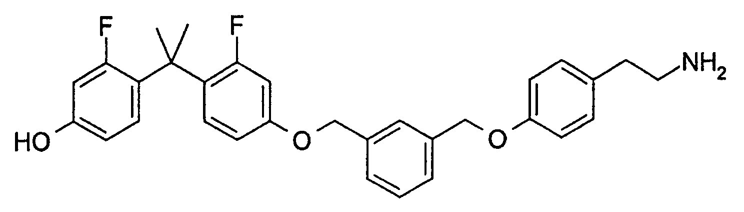 in the form of the free bases or the corresponding acid addition salts with pharmacologically acceptable acids.

4. Process for preparing compounds of general formula 1, **characterised in that** a compound of formula (2) 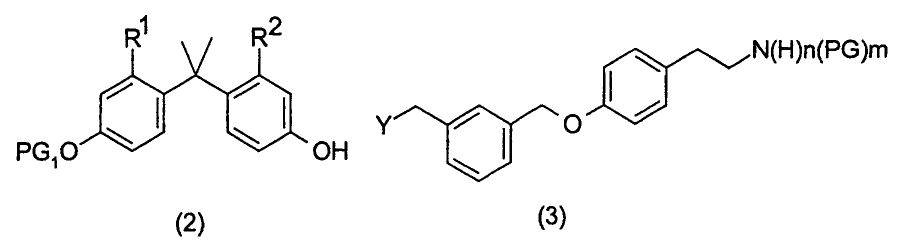 is reacted with an ethylamine derivative of general formula 3 wherein
PG₁ may denote a protecting group suitable for protecting phenols, or hydrogen;
PG may denote a protecting group suitable for protecting amines, where n is 0 or 1 and m may denote the integer 1 or 2;
Y may denote fluorine, chlorine, bromine or iodine or a C₁₋₄-alkyl or an arylsulphonate group,
and R¹ and R² are as hereinbefore defined
in the presence of an auxiliary base selected from among the alkali or alkaline earth metal hydroxides, alkali or alkaline earth metal carbonates, C₁₋₄-alkali metal alkoxides in solvents which are inert under the reaction conditions chosen, such as formamides, preferably dimethylformamide (DMF), C₁₋₄-alkylnitriles, preferably acetonitrile, C₁₋₄-alkyl esters of carboxylic acids, preferably ethyl acetate or ethyl formate, aromatic or aliphatic hydrocarbons, preferably toluene, or in branched or unbranched C₁₋₄-alcohols, in the following reaction step the protecting groups are cleaved using other methods known from the prior art and the reaction product is isolated.

5. Process for preparing compounds of general formula 1, **characterised in that** a compound of general formula 4 is reacted with a compound of formula 5 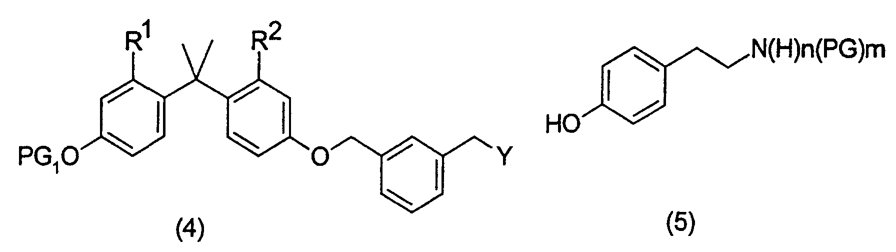 wherein
PG₁ may denote hydrogen or a protecting group suitable for protecting phenols;
PG may denote a protecting group suitable for protecting amines, where n is 0 or 1 and m may denote the integer 1 or 2;
Y may denote fluorine, chlorine, bromine or iodine or a C₁₋₄-alkyl or an arylsulphonate group,
and R¹ and R² are as hereinbefore defined;
in the presence of an auxiliary base selected from among the alkali or alkaline earth metal hydroxides, alkali or alkaline earth metal carbonates, C₁₋₄-alkali metal alkoxides in solvents which are inert under the reaction conditions chosen, such as formamides, preferably dimethylformamide (DMF), C₁₋₄-alkylnitriles, preferably acetonitrile, C₁₋₄-alkyl esters of carboxylic acids, preferably ethyl acetate or ethyl formate, aromatic or aliphatic hydrocarbons, preferably toluene, or in branched or unbranched C₁₋₄-alcohols, and in the following reaction step the protecting groups are cleaved, preferably with inorganic or organic acids in suitable solvents or by hydrogenolysis using methods known from the prior art.

6. Process for preparing compounds of general formula 1, **characterised in that** a nitrostyrene of general formula 6 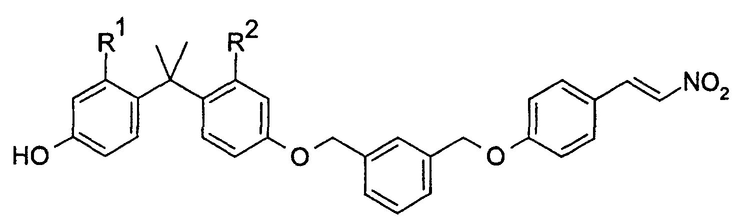 is reduced in a solvent selected from among methanol, ethanol or a higher alcohol, DMF or water in the presence of a catalyst from the group comprising Raney nickel, Pd/C, platinum or with hydride reagents, particularly complex hydrides selected from among NaBH₄, Ca(BH₄)₂, LiAlH₄ or other aluminium or boron hydrides, at temperatures in the range from 0 to 100°C and under a hydrogen pressure of more than 760 Torr (1.013 bar) to yield the corresponding amine of general formula 1.

7. Pharmaceutical preparation containing a compound of general formula 1 according to one of claims 1, 2 or 3 and the acid addition salts thereof together with conventional excipients and carriers.

8. Compound of formula 1 according to one of claims 1, 2 or 3 for use as a pharmaceutical composition.

9. Compound of formula 1 according to one of claims 1, 2 or 3 for use as a pharmaceutical composition having an LTB₄-antagonistic activity.

10. Use of compounds of general formula I, the stereoisomers and the acid addition salts thereof for preparing a medicament for the therapeutic treatment of arthritis, asthma, chronic obstructive lung diseases such as chronic bronchitis, psoriasis, ulcerative colitis, gastropathy or enteropathy induced by nonsteroidal antiinflammatories, cystic fibrosis, Alzheimer's disease, shock, reperfusion damage/ischaemia, atherosclerosis and multiple sclerosis.

## Revendications

1. Dérivés de phényléthylamine correspondant à la formule générale 1 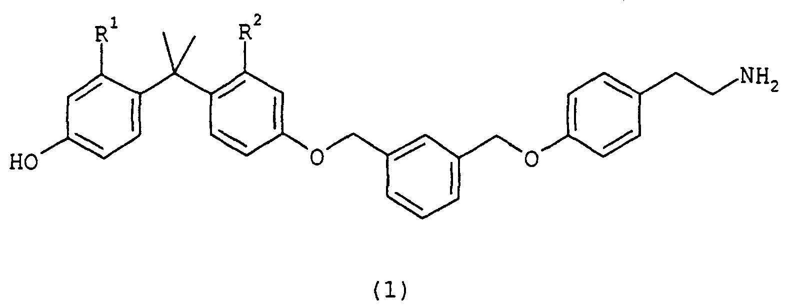 où
R¹ et R² peuvent représenter indépendamment l'un de l'autre l'hydrogène ou le fluor
sous forme des bases libres ou des sels d'addition d'acide correspondants avec des acides pharmacologiquement acceptables.

2. Dérivé de phényléthyléthylamine selon la revendication 1 de formule 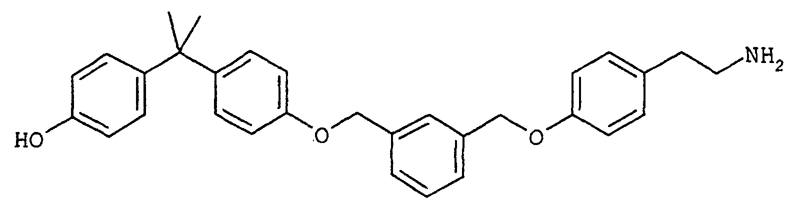 sous forme des bases libres ou des sels d'addition d'acide correspondants avec des acides pharmacologiquement acceptables.

3. Dérivé de phényléthyléthylamine selon la revendication 1 de formule 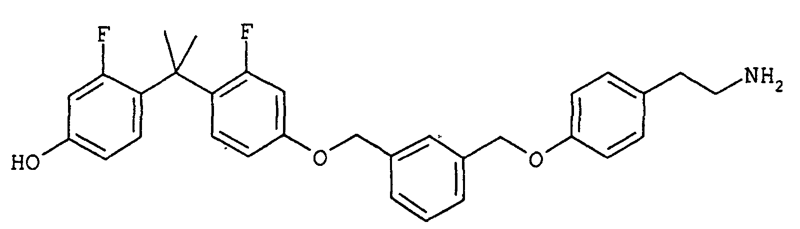 sous forme des bases libres ou des sels d'addition d'acide correspondants avec des acides pharmacologiquement acceptables.

4. Procédé de préparation de composés de formule générale 1 **caractérisé en ce que** l'on fait réagir un composé de formule (2) 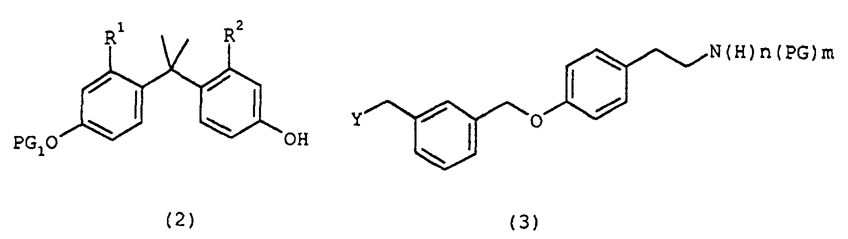 avec un dérivé d'éthylamine de formule générale (3) où PG₁ peut représenter un groupe protecteur approprié pour la protection de phénols ou l'hydrogène ;
PG peut représenter un groupe protecteur approprié pour la protection d'amines, où n peut représenter 0 ou 1 et m peut représenter l'un des nombres entiers 1 et 2 ;
Y peut représenter le fluor, le chlore, le brome ou l'iode ou un reste alkyle en C₁-C₄ ou un reste arylsulfonate
et R¹ et R² ont la signification citée au début
en présence d'une base auxiliaire du groupe des hydroxydes alcalins ou alcalino-terreux, des carbonates alcalins ou alcalino-terreux, des alcoolates alcalins en C₁-C₄ dans des solvants inertes dans les conditions réactionnelles choisies comme des formamides, de préférence le diméthylformamide (DMF), des alkyle en C₁-C₄-nitriles, de préférence l'acétonitrile, des esters d'alkyle en C₁-C₄ d'acides carboxyliques, de préférence l'acétate d'éthyle ou le formiate d'éthyle, des hydrocarbures aromatiques ou aliphatiques, de préférence le toluène, ou dans des alcools en C₁-C₄ ramifiés ou non ramifiés, dans l'étape réactionnelle suivante on clive les groupes protecteurs par d'autres procédés connus par l'état de la technique et on isole le produit réactionnel.

5. Procédé de préparation de composés de formule générale 1 **caractérisé en ce que** l'on fait réagir un composé de formule générale 4 avec un composé de formule 5 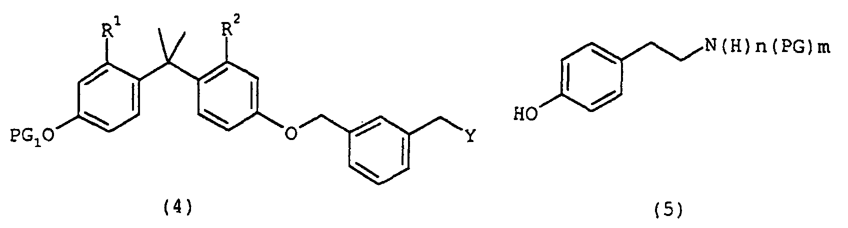 PG₁ peut représenter l'hydrogène ou un groupe protecteur approprié pour la protection de phénols ;
PG peut représenter un groupe protecteur approprié pour la protection d'amines, où n peut représenter 0 ou 1 et m peut représenter l'un des nombres entiers 1 et 2 ;
Y peut représenter le fluor, le chlore, le brome ou l'iode ou un reste alkyle en C₁-C₄ ou un reste arylsulfonate
et R¹ et R² ont la signification citée au début ;
en présence d'une base auxiliaire du groupe des hydroxydes alcalins ou alcalino-terreux, des carbonates alcalins ou alcalino-terreux, des alcoolates alcalins en C₁-C₄ dans des solvants, inertes dans les conditions réactionnelles choisies, comme des formamides, de préférence le diméthylformamide (DMF), des alkyle en C₁-C₄-nitriles, de préférence l'acétonitrile, des esters d'alkyle en C₁-C₄ d'acides carboxyliques, de préférence l'acétate d'éthyle ou le formiate d'éthyle, des hydrocarbures aromatiques ou aliphatiques, de préférence le toluène, ou dans des alcools en C₁-C₄ ramifiés ou non ramifiés, et dans l'étape réactionnelle suivante on clive les groupes protecteurs, de préférence avec des acides inorganiques ou organiques dans des solvants appropriés ou par hydrogénolyse au moyen de procédés connus en soi.

6. Procédé de préparation de composés de formule générale 1 **caractérisé en ce que** l'on réduit un nitrostyrène de formule générale 6 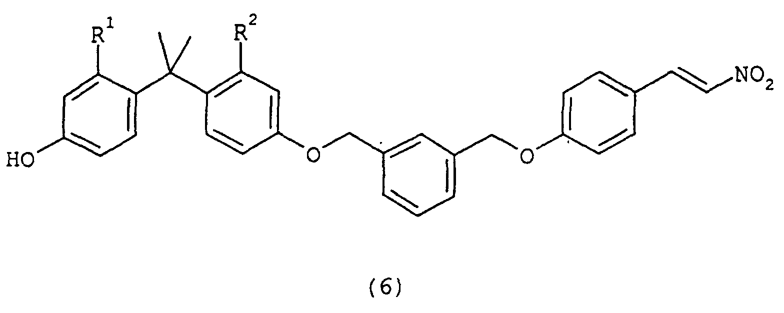 dans un solvant du groupe du méthanol, de l'éthanol ou dans un alcool supérieur, le DMF ou l'eau en présence d'un catalyseur du groupe du nickel de Raney, de Pd/C, du platine ou avec des réactifs hydrures, en particulier des hydrures complexes du groupe de NaBH₄, Ca(BH₄)₂, LiAlH₄ ou d'autres hydrures d'aluminium ou de bore, à des températures dans un intervalle de 0 à 100°C et sous une pression d'hydrogène supérieure à 760 torr (1,013 bar) en l'amine de formule générale 1 correspondante.

7. Préparation pharmaceutique contenant un composé de formule générale 1 selon l'une des revendications 1, 2 et 3 et ses sels d'addition d'acide, outre des adjuvants et supports courants.

8. Composé de formule 1 selon l'une des revendications 1, 2 et 3 destiné à être utilisé comme médicament.

9. Composé de formule 1 selon l'une des revendications 1, 2 et 3 destiné à être utilisé comme médicament à effet antagoniste de LTB₄.

10. Utilisation de composés de formule générale 1, de leurs stéréoisomères ainsi que de leurs sels d'addition d'acide pour la production d'un médicament pour le traitement thérapeutique de l'arthrite, de l'asthme, d'une maladie pulmonaire obstructive chronique comme la bronchite chronique, du psoriasis, de la colite ulcéreuse, d'une gastro- ou entéropathie induite par des antiphlogistiques non stéroïdiens, de la fibrose kystique, de la maladie d'Alzheimer, du choc, des lésions de reperfusion/ischémies, de l'athérosclérose, de la sclérose en plaques.
